# FASCICULE DE BREVET EUROPEEN

(11) **EP 0 399 417 B1**
(45) Date de publication et mention de la délivrance du brevet: **12.10.1994**
(21) Numéro de dépôt: 90109553.9
(22) Date de dépôt: 19.05.1990
(51) Int. Cl.: C07C 51/42, C11C 1/00

(54) **Préparation de l'acide stéaridonique**
Herstellung von Stearidonsäure
Preparation of stearidonic acid

(30) Priorité: 22.05.1989 CH 1919/89
(43) Date de publication de la demande: 28.11.1990
(73) Titulaire: SOCIETE DES PRODUITS NESTLE S.A., 1800 Vevey (CH)
(72) Inventeur: Lagarde, Michel, F-69150 Chassieu (FR); Traitler, Helmut, CH-1800 Vevey (CH); Wille, Hans-Juergen, CH-1844 Villeneuve (CH)

(56) Documents cités:
- EP-A- 0 271 747
- JOURNAL OF THE AMERICAN OIL CHEMISTS' SOCIETY, vol. 65, no. 5, mai 1988, Champaign, US, pages 755-760; H. TRAITLER et al: "Fractionation of black currant seed oil"
- REVUE FRANCAISE DES CORPS GRAS, vol. 34, no. 2, février 1987, Paris, FR, pages 69-74; H. J. WILLE et al: "Production of polyenoic fish oil fatty acids by combined urea fractionation and industrial scale preparative HPLC"

## Description

L'invention se rapporte à la préparation de l'acide stéaridonique à partir d'un mélange d'acides gras le contenant comme constituant mineur.

On sait que les acides gras essentiels de la série n-3, (nomenclature définie par l'emplacement de la première double liaison à partir du groupe méthyl) aboutissent par une chaîne réactionnelle aux prostaglandines de la série 3 inhibitrices de l'agrégation des plaquettes sanguines.

Le premier élément de cette chaîne est l'acide alpha-linolénique (ALA, C 18:3 Δ 9, 12, 15) dont la transformation en acide stéaridonique (SA, C 18:4 Δ 6, 9, 12, 15) est tributaire de l'activité de l'enzyme Δ 6 désaturase dont on sait qu'elle diminue lors du vieillissement et à la suite de certaines maladies. La synthèse des prostaglandines de la série 3 s'en trouve compromise. Dans le but de remédier à cette faiblesse de l'organisme, on propose d'introduire directement dans le corps l'acide stéaridonique.

La demande de brevet Japonais No 86050752 concerne un procédé de séparation de l'acide stéaridonique de l'huile de poisson consistant à transformer les acides gras qu'elle contient en leurs esters éthyliques, à traiter les esters éthyliques par distillation moléculaire et à recueillir la fraction de tête, puis à la faire réagir avec l'urée dans le méthanol, à recueillir la fraction n'ayant pas réagi et enfin à séparer une fraction enrichie en ester éthylique de l'acide stéaridonique par deux opérations successives de chromatographie de partition en phase renversée.

On obtiendrait de cette manière une pureté de 85% en poids avec un rendement de 1,8% par rapport à l'huile mise en oeuvre, ce qui représente une récupération de 48% de l'acide stéaridonique présent dans la matière première.

Par ailleurs, le brevet européen No 178.442 concerne l'enrichissement de l'huile de pépins de cassis en acide gamma-linolénique (GLA, C 18:3 Δ 6, 9, 12) par double complexation à l'urée d'un mélange d'acides gras provenant de la saponification de l'huile de cassis, suivie d'une chromatographie liquide à haute performance en phase renversée. D'après ce brevet, la séparation paraît particulièrement difficile, l'enrichissement du mélange ne paraissant possible qu'en ce qui concerne son constituant majeur, l'acide gamma-linolénique.

Nous avons trouvé qu'on pouvait préparer une fraction enrichie en acide stéaridonique d'une pureté supérieure à 90% en poids à partir d'un mélange d'acides gras le contenant sans recourir à la distillation moléculaire des esters éthyliques et avec une seule opération chromatographique.

L'invention concerne donc un procédé de préparation de l'acide stéaridonique, dans lequel on fait réagir un mélange d'acides gras contenant cet acide avec l'urée et on recueille une fraction enrichie en acides gras polyinsaturés, puis on injecte la fraction enrichie dans une colonne de chromatographie liquide haute performance en phase renversée et on l'élue de cette colonne.

Le procédé selon l'invention est caractérisé par le fait que l'on utilise comme éluant un mélange pondéral de 75 à 95% de méthanol pour 25 à 5% d'eau et que l'on injecte les acides gras dans la colonne sous forme d'une solution à 25 à 35% en poids.

Pour mettre en oeuvre le procédé de l'invention, on utilise de préférence une huile de départ contenant un pourcentage appréciable d'acide stéaridonique, de l'ordre de 2 à 4%. L'huile de poisson et particulièrement l'huile de pépins de cassis (Ribes nigrum) sont à cet égard appropriées.

L'huile de poisson contient des proportions intéressantes des acides stéaridonique, éicosapentaénoique (EPA, C 20:5 Δ 5, 8, 11, 14, 17) et docosahexaénoique (DHA, C 22:6 Δ 4,7, 10, 13, 16, 19), cependant que l'huile de pépins de cassis constitue une source importante des acides gamma-linolénique et stéaridonique.

On raffine tout d'abord les huiles brutes extraites par exemple par solvant ou par pression en procédant de manière connue à leur dégommage, par exemple au moyen d'acide phosphorique, à la neutralisation des acides gras libres par exemple par la soude, à leur décoloration par exemple à l'aide d'un mélange de charbon actif et de silicate d'aluminium activé, à leur désodorisation sous vide, à une température d'environ 200°C dans le cas de l'huile de pépins de cassis, puis le cas échéant à leur winterisation en refroidissant par exemple à environ 4°C pendant environ 24 h, ce qui permet d'éliminer la plupart des cires résiduelles dans le cas de l'huile de pépins de cassis.

Le raffinage de l'huile de poisson s'opère comme pour l'huile de pépins de cassis, mais la désodorisation s'effectue de préférence à une température ≦ 180°C pour éviter la dégradation des acides gras polyinsaturés. On obtient le mélange d'acides gras libres de départ par saponification des huiles raffinées ou semi-raffinées, puis acidification des sels d'acides gras obtenus ou encore par hydrolyse des huiles raffinées ou semi-raffinées. Le semi-raffinage comprend le dégommage puis la neutralisation des huiles brutes.

On traite ensuite le mélange d'acides gras par l'urée dans un rapport pondéral acides gras:urée de 1:3 à 1:6, de préférence 1:3 à 1:4, en présence de méthanol dans un rapport pondéral urée:méthanol de 1:1,5 à 1:3 et de préférence 1:2,1 en chauffant le mélange jusqu'à l'ébullition. On refroidit le mélange réactionnel jusqu'à une température allant de -5 à 20°C, et de préférence de 0 à 15°C, puis on laisse le mélange refroidi à la température finale durant au plus 15 h, et de préférence pendant 1 à 7 h. On filtre ensuite à froid le précipité formé et on récupère la phase liquide dont on extrait le mélange d'acides gras enrichi en polyinsaturés, par exemple dans un milieu hydrochlorique en présence d'hexane, dont on élimine ensuite l'hexane.

L'étape suivante est le fractionnement du mélange d'acides gras enrichi en polyinsaturés par chromatographie liquide à haute performance en phase renversée. Ce type de chromatographie préparative consiste à injecter les acides gras dans l'appareil sous forme d'échantillons, à les adsorber par leur partie hydrophobe sur une colonne constituant la phase stationnaire composée de silice poreuse dopée d'une couche d'hydrocarbures saturés, puis à les désorber sélectivement par une phase mobile de polarité contrôlée dans laquelle sont dissoutes les parties polaires des échantillons et qui constitue l'éluant.

Dans le cas présent, l'éluant est composé d'un mélange hydro-méthanolique contenant de préférence en poids, 85 à 90% de méthanol pour 15 à 10% d'eau. La phase mobile représente 1 à 3 g par litre d'adsorbant dans la colonne et de préférence 1,5 à 2 g/l. Les acides gras se présentent dans l'échantillon d'injection de préférence sous forme d'une solution dans un bon solvant de ceux-ci, par exemple le méthanol. La concentration des acides gras dans l'échantillon d'injection est un facteur déterminant de réussite de la séparation. Elle est de préférence d'environ 30% en poids pour environ 70% en poids de méthanol. De fait, il s'est avéré que la séparation de l'acide stéaridonique est plus performante avec une solution plus concentrée d'acides gras que dans le cas des séparations par chromatographie de l'état de la technique. L'influence de la concentration dans cette direction est inattendue en ce sens qu'on pouvait s'attendre à ce que la séparation soit facilitée par une dilution. Le résultat remarquable du procédé selon l'invention est dû principalement au choix approprié de la composition de l'éluant et de la concentration en acides gras de la phase mobile.

L'invention concerne également l'utilisation de l'acide stéaridonique obtenu par le procédé selon l'invention pour fabriquer une composition pharmaceutique contre les maladies cardiovasculaires et thrombo-emboliques associées à l'agrégation plaquettaire.

Une composition pharmaceutique précédente peut se présenter sous différentes formes adaptées au mode d'administration par exemple par voie orale, entérale, rectale ou parentérale. On peut par exemple préparer des capsules, gélules, suppositoires ou sirops. Dans le cas d'une administration entérale ou parentérale, les compositions se présentent sous forme de solutions ou émulsions stabilisées chimiquement et physiquement, apyrogènes et stériles.

L'acide stéaridonique peut être avantageusement protégé de l'oxydation par un antioxydant approprié, par exemple le palmitate d'ascorbyle, les tocophérols, un mélange de tels antioxydants ou un mélange d'acide ascorbique, de tocophérol et de lécithine.

La dose administrée dépend du type et du degré de la maladie à traiter. Elle peut être de 0,1 à 1 g d'acide stéaridonique par jour en prise unique ou de préférence en 2 à 3 prises séparées.

Les exemples ci-après illustrent l'invention. Dans ceux-ci, les parties et pourcentages sont pondéraux sauf indication contraire.

Dans ces exemples, l'analyse des acides gras a lieu par chromatographie en phase gazeuse de leurs esters méthyliques.

### Exemple 1

1.1. A 100 parties d'huile de pépins de cassis raffinée on ajoute une solution de 30,5 parties d'hydroxyde de sodium dans un mélange de 101,5 parties d'eau et 80,5 parties d'éthanol sous bonne agitation et l'on chauffe le mélange jusqu'à l'ébullition (environ 80°C) durant 30 min. On refroidit ensuite le mélange à 30-40°C et on y ajoute lentement 100 parties d'acide chlorhydrique à 32% (de pH 1) et 166,7 parties d'hexane sous agitation vigoureuse durant 30 min. On laisse alors décanter le mélange. Il se forme deux phases. On prélève la phase supérieure dont on évapore l'hexane à 40°C sous vide et qui constitue le mélange d'acides gras de départ. Ce mélange est composé des acides gras suivants:

| | % |
|---|---|
| C 16:0 | 7 |
| C 18:0 | 1,3 |
| C 18:1 cis | 11,7 |
| C 18:2 Δ 9, 12 | 47,3 |
| C 18:3 Δ 6, 9, 12 | 16,4 |
| C 18:3 Δ 9, 12, 15 | 13,8 |
| C 18:4 Δ 6, 9, 12, 15 (SA) | 2,5 |

1.2 On ajoute 450 g d'urée et 945 g de méthanol à 150 g du mélange d'acides gras précédent, puis on chauffe le mélange jusqu'à ébullition où il devient limpide. On le laisse alors refroidir à la température ambiante puis on le place dans un bain-marie à 0°C pendant 5 h. Il se forme un précipité. On récupère ensuite la phase liquide par filtration sous vide sur Büchner préalablement refroidi à 0°C, puis on en extrait les acides gras enrichis en polyinsaturés au moyen de 0,2 partie d'acide chlorhydrique, 0,6 partie d'eau et 0,25 partie d'hexane pour 1 partie de phase liquide. Enfin, on évapore l'hexane à 40°C sous vide et on recueille un mélange d'acides gras de composition ci-après:

| | % |
|---|---|
| C 18:2 Δ 6, 9 | 2,1 |
| C 18:3 Δ 6, 9, 12 | 80,7 |
| C 18:3 Δ 9, 12, 15 | 2,1 |
| C 18:4 Δ 6, 9, 12, 15 (SA) | 15,1 |

1.3 La chromatographie s'effectue au moyen de deux colonnes disposées en série de 5,7 cm de diamètre intérieur et 30 cm de longueur garnies de silice poreuse dopée d'hydrocarbures saturés en C 18 constituant la phase stationnaire. Les colonnes sont sous pression d'azote à 30 bar (compression radiale) et sont munies d'un détecteur référentiel d'indice de réfraction permettant l'identification quantitative des fractions. La phase mobile est constituée d'un mélange méthanol:eau à environ 12% d'eau, de densité 0,821-0,824. On injecte des échantillons de 10 ml d'une solution de 30% du mélange d'acides gras dans 70% de méthanol de manière à les adsorber sur la colonne. On élue ensuite les différentes fractions en faisant passer la phase mobile à travers la colonne à un débit de 100 ml/min. On recueille plusieurs fractions et on réserve celle relative au premier pic. Celle-ci correspond à 10% du mélange d'acides gras introduit dans l'appareil et contient 92% d'acide stéaridonique. Le rendement en acide stéaridonique de pureté 92% se monte ainsi à 1,5% du mélange d'acides gras de départ, ce qui correspond à 56% de l'acide stéaridonique contenu dans la matière première.

### Exemple 2

On réinjecte la fraction obtenue à l'exemple 1 sous forme d'une solution à 30% dans le méthanol en échantillon de 10 ml dans les mêmes conditions. On recueille la fraction relative au premier pic correspondant à 60% du mélange d'acides gras réinjecté qui contient 98% d'acide stéaridonique.

### Exemple 3

On procède comme à l'exemple 1 à partir d'un mélange d'acides gras d'huile de poisson fractionné à l'urée dans les mêmes conditions mis à part la température finale de repos du mélange complexe avec l'urée / phase liquide qui est de 10°C. Le mélange d'acides gras a la composition ci-après:

| | % |
|---|---|
| C 18:4 Δ 6, 9, 12, 15 (SA) | 16,3 |
| C 20:5 Δ 5, 8, 11, 14, 17 (EPA) | 34,9 |
| C 22:6 Δ 4, 7, 10, 13, 16, 19 (DHA) | 38,6 |
| Autres | 10,2 |

On conduit la chromatographie comme à l'exemple 1 en ce qui concerne la composition de la phase mobile et le débit d'élution. Seule change la charge spécifique en acides gras de départ de 1,75 g/l d'adsorbant dans la colonne. On recueille plusieurs fractions et on réserve celle relative au second pic qui a la composition suivante en acides gras:

| | % |
|---|---|
| C 18:3 Δ 9, 12, 15 (ALA) | 5 |
| C 18:4 Δ 6, 9, 12, 15 (SA) | 86 |
| Autres | 9 |

Cette fraction représente 0,9% du mélange d'acides gras de départ, ce qui correspond à 35% de l'acide stéaridonique contenu dans la matière première.

### Exemple 4

Il est admis que les maladies cardiovasculaires et thrombo-emboliques sont souvent associées à une hyperactivité des plaquettes sanguines. Les acides gras polyinsaturés de la série n-3 sont considérés comme des facteurs de prévention des maladies thrombo-emboliques.

On étudie l'effet de l'acide stéaridonique obtenu selon l'exemple 2 sur l'agrégation plaquettaire comparativement à ceux des acides gamma-linolénique, alpha-linolénique, eicosapentaénoïque ainsi que du mélange d'acides gras obtenu selon l'exemple 2 du brevet EP 178.442 (ci-après concentrat).

4.1 On recueille le sang de donneurs sains de moyenne d'âge 36 ans auquel on ajoute un anticoagulant et on en isole les plaquettes. On met ensuite les plaquettes en suspension dans une solution Tyrode HEPES de pH 7,35 gardée 16 h à 37°C dans des tubes fermés sous azote et contenant:
50 µM d'albumine humaine délipidée,
50 µM d'acide linoléique (LA, C 18:2 Δ 9, 12)
5 µM d'acide arachidonique (AA, C 20:4 Δ 5, 8, 11, 14)
et 5 µM de l'acide gras étudié.

On incube les plaquettes dans cette solution pendant 2 h à 37°C, puis on les isole à nouveau et on les reprend dans une solution Tyrode HEPES à laquelle on ajoute 0,1% de gélatine. Pour chaque essai, un lot de suspension plaquettaire servant de témoin subit le même traitement sauf que la suspension ne contient que les acides linoléique et arachidonique dont l'apport ne modifie pas les fonctions plaquettaires. Dans certains essais on ajoute encore 10 µM d'alpha-tocophérol en plus des différents acides gras pour compenser la diminution de ce composé induite par les acides gras polyinsaturés. Les plaquettes ainsi enrichies se trouvent dans des conditions proches de celles régnant dans le plasma sanguin.

4.2 L'agrégation plaquettaire est mesurée selon la méthode turbidimétrique de Born (J. Physiol. 209:487, 1973).

La concentration de l'agent agrégant est déterminée pour obtenir environ 60% d'agrégation 4 min. après l'addition de l'agent agrégant, avec les plaquettes considérées comme témoin.

4.3 Les résultats sont indiqués dans le tableau 1 ci-après exprimés en % d'agrégation et représentent une moyenne ± la déviation standard chaque fois en parallèle avec le témoin.

**TABLEAU 1**

| Acide gras apporté | Collagène | Agent agrégant | | |
|---|---|---|---|---|
| | | Acide arachidonique | Analogue de la PGH2 (U 46619) | Thrombine |
| SA | 47 ± 12 | 30 ± 6 | 51 ± 15 | 53 ± 12 |
| Témoin | 58 ± 8 | 47 ± 5 | 63 ± 3 | 62 ± 7 |
| GLA | 51 ± 12 | - | 47 ± 11 | 57 ± 15 |
| Témoin | 60 ± 9 | - | 62 ± 11 | 59 ± 6 |
| ALA | 47 ± 12 | - | 50 ± 9 | 54 ± 3 |
| Témoin | 58 ± 9 | - | 56 ± 7 | 61 ± 10 |
| EPA | 46 ± 12 | - | 45 ± 13 | 59 ± 13 |
| Témoin | 60 ± 9 | - | 62 ± 11 | 59 ± 8 |
| Concentrat | 50 ± 9 | - | 57 ± 13 | 61 ± 8 |
| Témoin | 58 ± 9 | - | 57 ± 6 | 61 ± 10 |
| Légende : - = non testé | | | | |

Les résultats précédents montrent que le SA inhibe l'agrégation plaquettaire induite par le collagène, l'acide arachidonique, l'analogue de la PGH2 ou la thrombine. Comparé aux GLA, ALA et EPA, le SA inhibe plus spécifiquement la stimulation par la thrombine. Par ailleurs, on a constaté que la présence de 10 µM d'alpha-tocophérol ne modifiait pas l'agrégation plaquettaire.

## Revendications

1. Procédé de préparation de l'acide stéaridonique, dans lequel on fait réagir un mélange d'acides gras contenant cet acide avec l'urée et on recueille une fraction enrichie en acides gras polyinsaturés, puis on injecte la fraction enrichie dans une colonne de chromatographie liquide à haute performance en phase renversée et on l'élue de cette colonne, caractérisé par le fait que l'on utilise comme éluant un mélange pondéral de 75 à 95% de méthanol pour 25 à 5% d'eau et que l'on injecte les acides gras dans la colonne sous forme d'une solution à 25 à 35% en poids.

2. Procédé selon la revendication 1, caractérisé par le fait que le mélange d'acides gras de départ provient de l'huile de pépins de cassis (Ribes nigrum).

3. Procédé selon la revendication 1, caractérisé par le fait que le mélange d'acides gras de départ provient de l'huile de poisson.

4. Procédé selon la revendication 1, caractérisé par le fait que l'on utilise 1 à 3 g de phase mobile par litre d'adsorbant constituant la phase stationnaire dans la colonne.

5. Procédé selon la revendication 1, caractérisé par le fait que l'on réinjecte la fraction enrichie en acide stéaridonique dans la colonne et on recueille l'acide stéaridonique pratiquement pur.

6. Utilisation de l'acide stéaridonique obtenu par le procédé selon l'une des revendications 1 à 5 pour fabriquer une composition pharmaceutique contre les maladies cardiovasculaires et thrombo-emboliques associées à l'agrégation plaquettaire.

7. Utilisation selon la revendication 6, sous une forme adaptée à l'administration par voie orale, rectale, entérale ou parentérale.

8. Utilisation selon la revendication 6, sous une forme fournissant une dose quotidienne de 0,1 à 1 g d'acide stéaridonique.

## Claims

1. A process for the production of stearidonic acid in which a mixture of fatty acids containing this acid is reacted with urea and a fraction enriched with polyunsaturated fatty acids is collected, the enriched fraction is injected into a high-performance reverse-phase liquid chromatography column and is eluted from the column, characterized in that a mixture of 75 to 95% by weight methanol for 25 to 5% by weight water is used as eluent and the fatty acids are injected into the column in the form of a 25 to 35% by weight solution.

2. A process as claimed in claim 1, characterized in that the starting fatty acid mixture emanates from blackcurrant seed oil (Ribes nigrum).

3. A process as claimed in claim 1, characterized in that the starting fatty acid mixture emanates from fish oil.

4. A process as claimed in claim 1, characterized in that 1 to 3 g mobile phase is used per litre adsorbent constituting the stationary phase in the column.

5. A process as claimed in claim 1, characterized in that the fraction enriched with stearidonic acid is injected into the column and substantially pure stearidonic acid is collected.

6. The use of the stearidonic acid obtained by the process claimed in any of claims 1 to 5 for the production of a pharmaceutical composition against the cardiovascular and thrombo-embolic diseases associated with platelet aggregation.

7. The use claimed in claim 6 in a form suitable for oral, rectal, enteral or parenteral administration. 8. The use claimed in claim 6 in a form supplying a daily dose of 0.1 to 1 g stearidonic acid.

## Patentansprüche

1. Verfahren zur Herstellung von Stearidonsäure, bei dem man eine Fettsäuremischung, die diese Säure enthält, mit Harnstoff umsetzt und eine Fraktion gewinnt, die bezüglich mehrfach ungesättigter Fettsäuren angereichert ist, dann die angereicherte Fraktion in eine Säule für die Umkehrphasen-Hochleistungs-Flüssigkeitschromatographie injiziert und die Säule eluiert, dadurch gekennzeichnet, daß man als Elutionsmittel eine Mischung von 75 bis 95 Gew.-% Methanol und 25 bis 5 Gew.-% Wasser verwendet und man die Fettsäuren in Form einer 25 bis 35 Gew.-%igen Lösung in die Säule injiziert.

2. Verfahren nach Anspruch 2, dadurch gekennzeichnet, daß die Ausgangsmischung der Fettsäuren aus dem Öl von Kernen der schwarzen Johannisbeere (Ribes niger) stammt.

3. Verfahren nach Anspruch 1, dadurch gekennzeichnet, daß die Ausgangsmischung der Fettsäuren aus Fischöl stammt.

4. Verfahren nach Anspruch 1, dadurch gekennzeichnet, daß man pro Liter des Adsorptionsmittels, das die stationäre Phase der Säule bildet, 1 bis 3 g mobile Phase verwendet.

5. Verfahren nach Anspruch 1, dadurch gekennzeichnet, daß man die bezüglich der Stearidonsäure angereicherte Fraktion in die Säule reinjiziert und die Stearidonsäure in praktisch reiner Form gewinnt.

6. Verwendung der nach einem Verfahren nach einem der Ansprüche 1 bis 5 erhaltenen Stearidonsäure zur Herstellung einer pharmazeutischen Zusammensetzung gegen kardiovaskuläre und thrombembolische Erkrankungen, die mit einer Blutplättchenaggregation assoziiert sind.

7. Verwendung nach Anspruch 6 in einer für die orale, rektale, enterale oder parenterale Verabreichung geeigneten Form.

8. Verwendung nach Anspruch 6 in einer Form für die Zufuhr einer Tagesdosis von 0,1 bis 1 g Stearidonsäure.
